# EUROPEAN PATENT APPLICATION

(11) **EP 1 859 792 A1**
(43) Date of publication of application: **28.11.2007**
(21) Application number: 06380132.8
(22) Date of filing: 24.05.2006
(51) Int. Cl.: A61K 9/51, A61K 48/00

(54) **Nanoparticles of chitosan and hyaluronan for the administration of active molecules**

(71) Applicant: Advanced in Vitro Cell Technologies, S.L., 08028 Barcelona (ES)
(72) Inventor: Alonso Fernandez, M. José, 15782 Santiago de Compostela (ES); De la Fuente Freire, Maria, 15782 Santiago de Compostela (ES); Seijo Rey, Begoña, 15782 Santiago de Compostela (ES); Vila Pena, Ana Isabel, Lab. Feder 15782 Santiago de Compostela (ES)
(74) Representative: ABG Patentes, S.L.

(57) **Abstract**

The present invention relates to a nanoparticulate system useful for the delivery of pharmacologically active molecules, and especially for transfecting polynucleotides into cells. It comprises nanoparticles of chitosan of low molecular weight and hyaluronan.

## Description

### FIELD OF THE INVENTION

The invention relates to a nanoparticulate system useful for the release of pharmacologically active molecules, and especially for transfecting polynucleotides into cells. It is aimed at systems which comprise nanoparticles of chitosan of low molecular weight and hyaluronan, and pharmaceutical compositions which comprise them, as well as processes for their preparation.

### BACKGROUND OF THE INVENTION

The administration of biologically active molecules for therapeutic purposes presents numerous difficulties, both depending on the route of administration and the physicochemical and morphological characteristics of the molecules. It is known that the main drawbacks arise when administering unstable or large-sized active molecules. Access of macromolecules to the interior of the organism is limited by the low permeability of the biological barriers. Likewise, they are susceptible of being degraded due to the different defense mechanisms that both human and animal organisms have.

It has been demonstrated that the incorporation of macromolecules in nanometric-sized systems makes it easier for them to penetrate the epithelial barriers and protects them from being degraded. Thus, the design of nanoparticulate systems capable of interacting with said barriers is presented as a promising strategy in order to achieve the penetration of active ingredients through mucous membranes.

It is also known that the capacity of these systems to cross external barriers and access the interior of the organism, both depends on their size and on their composition. Small-sized particles will increase the degree of transport with respect to those of larger size; nanoparticles, with diameter less than 1 µm, respond to this criteria. If they are prepared from polymers of natural and biocompatible origin, the possibilities increase of them being naturally transported through the organism's mucous membranes, by known transport mechanisms and without altering the epithelial physiology.

In this sense, chitosan has been used as natural and biocompatible polymer in the formulation of nanoparticulate systems (WO-A-01/32751, WO-A-99/47130, ES 2098188) due to the fact that it provides a positive charge to the nanoparticles which allows the absorption through a biological environment of anionic character and/or its adhesion to negatively charged biological membranes.

Another polymer used in the elaboration of these systems is the hyaluronic acid, a natural polymer which is present in the extracellular matrix of connective tissues as well as in the vitreous body of the ocular globe and in the synovial fluid of articular cavities. It is a biodegradable and biocompatible polymer, which is not immunogenic and has mucoadhesive properties. Additionally, the hyaluronan interacts with the CD44 receptor which is present in the majority of organism cells.

Thus, document WO89/03207 and the article by Benedetti et al., Journal of Controlled Release 13, 33-41 (1990) show the production of hyaluronic acid microspheres according to a solvent evaporation method. Also, document US6,066,340 relates to the possibility of obtaining said microspheres making use of solvent extraction techniques.

Document US2001053359 proposes the combination, for nasal administration, of an antiviral and a bioadhesive material, being presented in the form of a solution or microspheres comprised of different materials, among others, gelatine, chitosan or hyaluronic acid, but not mixtures thereof. The microparticles are obtained by classic techniques such as atomising and solvent emulsion/evaporation. Once obtained, the microparticles are hardened by conventional chemical crosslinking methods (dialdehydes and diketones).

The combination of chitosan (or other cationic polymers) and hyaluronic acid has been proposed in micro- and nanoparticulated systems with the aim of combining the mucoadhesive effect of hyaluronic acid with the absorption promoting effect of the chitosan and to improve the interaction and absorption of nanoparticles with epithelial barriers. The value of this microparticulate combination is reflected in the works by Lim et al., J. Controll. Rel. 66, 2000, 281-292 and Lim et al., Int. J. Pharm. 23, 2002, 73-82. As with the previous document, these microparticles have been prepared by the solvent emulsion-evaporation technique.

Document US6,132,750 relates to the preparation of small-sized particles (micro and nanoparticles) which contain at least one protein (collagen, gelatine) and to a polysaccharide (chitosan or glycosaminoglycans such as hyaluronic acid, among others) on their surface. They are formed by interfacial crosslinking with a polyfunctional acylating agent which forms amide or ester bonds, and optionally anhydrous bonds.

Document WO2004/112758 refers to a nanoparticulate system for the administration of active molecules wherein the nanoparticles are constituted by a reticulated conjugate comprising a cationic polymer such as chitosan with a molecular weight of 125-150 kDa, collagen or gelatine and hyaluronic acid salt. They are formed by electrostatic interaction between both polymers which presents different charge and by ionotropic crosslinking in the presence of a crosslinking agent.

However, the main drawback associated to these systems is its low biodegradability in the biological environment, once the nanoparticles have been incorporated therein. Although it is well known that the hyaluronic acid or its corresponding salts are effectively degraded inside the cells by hyaluronidase enzimes, the chitosan biodegradability is highly questioned and the delivery of the active molecules is not as efficient as required. This fact can reduce considerably the effectiveness of the liberation of the active molecule present in the nanoparticles.

Additionally, the chitosan used in these systems is not soluble at pH higher than 6.6-6.8, which reduces its applicability when administering biologically active molecules, such as DNA.

In the case of transfection of nucleotide molecules such as DNA and RNA to cell for gene therapy, delivery systems are needed that can efficiently introduce the molecules into the target cells, with as low toxicity as possible and high transfection efficiency. It is important that the molecule introduced is adequately liberated and that it performs its function as efficiently as possible. The system comprising these molecules should be stable and easily administrable, in order to be accepted by the patient.

It would therefore be highly desirable to find a system for the release of active ingredients which allow a very efficient incorporation into the biological system, a rapid biodegradability of the nanoparticles in the biological environment. Further these systems should be easily produced and stable upon storage and transportation.

In the particular case of gene therapy, systems are needed that are as efficient as possible concerning the transfection of the target cells, and convenient for the patient.

### BRIEF DESCRIPTION OF THE INVENTION

The inventors have found that a system comprising nanoparticles that comprise chitosan and hyaluronan, wherein the molecular weight of the chitosan is less than 90 kDa, allows, in addition to an efficient association of biologically active molecules, an effective and easy degradation of the nanoparticles in the biological environment, thus favouring the release of the active molecules therein. It has been observed with *in vitro* studies that the system of the invention enables the efficient internalization of the nanoparticles in the cells due to cellular endocytosis processes and also to the interaction with specific receptors of the cellular membrane.

Additionally, *in vivo* studies have demonstrated the capacity of the nanoparticles of the invention to enter the epithelial cells, for example the corneal epithelial cells, and to deliver DNA-plasmid in a very effective manner, thus reaching important transfection levels, which make the system of the invention a new strategy towards gene therapy of several diseases. This transfection efficiency is also observed even when nanoparticles have been previously subjected to a freeze-drying process, which makes possible that the system of the invention can be stored without affecting its properties and stability.

The nanoparticulate system of the invention is surprisingly stable at pH between 6.4 and 8.0, depending on the composition, which makes it very versatile for different modalities of administration, including nasal, oral administration and topical application, and which ensures the stability of the nanoparticles at the plasma pH of 7.4. This stability is also of prime importance for transfecting cell cultures in applications "*in vitro*".

Thus, an object of the present invention consists of a system for the release of biologically active molecules which comprises nanoparticles with an average size less than 1 micrometer, wherein the nanoparticles comprise:
a) hyaluronan or a salt thereof; and
b) chitosan or a derivative thereof,
characterized in that the molecular weight of the chitosan or the derivative thereof is less than 90 kDa.

A second object of the invention refers to a such as described above which further comprises a biologically active molecule selected from the group consisting of polysaccharides, proteins, peptides, lipids, oligonucleotides, polynucleotides, nucleic acids and mixtures thereof.

A third object of the invention relates to a pharmaceutical composition which comprises a system such as defined above and a biologically active molecule capable of preventing, relieving or curing diseases.

Another object of the invention relates to a pharmaceutical composition as described above for gene therapy.

Another object of the invention relates to a process for the preparation of a system defined above, which comprises:
a) preparing an aqueous solution of hyaluronan;
b) preparing an aqueous solution of chitosan or a derivative thereof;
c) adding a crosslinking agent to the aqueous solution of the hyaluronan; and
d) mixing, under stirring, solutions obtained in b) and c) with spontaneous formation of the nanoparticles;
wherein, optionally, a biologically active molecule is dissolved in any of the previous aqueous solutions b) or c) before the formation of the nanoparticles, or it is dissolved in the nanoparticles suspension obtained in step d).

Finally, another object of the invention refers to the use of a system such as defined above in the preparation of a medicament for gene therapy.

### BRIEF DESCRIPTION OF THE DRAWINGS

**Figure 1:** Encapsulation efficiency of plasmid pGFP in the nanoparticulate system.
**Figure 2**: Sustained in vitro release of plasmid pGFP. Polymer mass proportion HA:CS or HA:CSO 1:2; time points: 0.5, 1, 4 and 24 h; chitosinase activity: 0.105 U.
**Figure 3:** In vitro cell toxicity when administering nanoparticles containing chitosan (CSO) and hyaluronate (HA) in a weight proportion 2:1 to three different cell lines (HEK 293, NHC and HCE).
**Figure 4:** In vitro cell toxicity using HEK 293 as cell line (1 h incubation) when administering nanoparticles containing chitosan (CSO) and hyaluronate (HA or HAO) in a weight proportion 2:1.
**Figure 5:** Confocal microscope images showing cellular transfection efficiency when delivering to HEK 293 cell line, DNA-plasmid pGFP from nanoparticles containing chitosan (CS or CSO) and hyaluronate (HA or HAO) in a weight proportion 2:1, after 2, 4, 6, 8 and 10 days.
**Figure 6:** Cellular transfection efficiency when delivering to HEK 293 cell line, DNA-plasmid pGFP from nanoparticles containing chitosan (CS or CSO) and hyaluronate (HA or HAO) in a weight proportion 1:1.
**Figure 7:** Confocal microscope images showing in vitro cell uptake after 1 hour and 12 hours post-incubation. Formulations: HAO:CSO; HAO:CS and HA:CS in a weight proportion 1:2, loaded with 1% of plasmid pGFP.
**Figure 8:** Confocal microscope images showing internalization of nanoparticles by cells using a cell line HCE at: a) 37°C; b) 4°C and c) 4°C blocking CD44 receptor with Ab Hermes 1. Formulation: HA:CSO in a weight proportion 1:2.
**Figure 9:** Confocal microscope images showing nanoparticles degradation in corneal epithelium as a function of time (2, 4 and 12 hours). Formulations: HA:CSO and HA:CS in a weight proportion 1:2.
**Figure 10:** Confocal microscope images showing the expression of encoded green protein in corneal epithelia of rabbits. Formulations: HA:CSO and HA:CS in a weight proportion 1:2 loaded with plasmid pEGFP.
**Figure 11:** Confocal microscope images showing cellular transfection efficiency when delivering to HEK 293 cell line, DNA-plasmid pEGFP from lyophilized nanoparticles containing chitosan (molecular weight of 14, 31 and 45 kDa) and hyaluronic acid in a weight proportion 1:2 and 2:1, after 4 days.

### DETAILED DESCRIPTION OF THE INVENTION

The system of the present invention comprises nanoparticles whose structure is a reticulate of a chitosan whose molecular weight is less than 90 kDa and hyaluronan, wherein a biologically active molecule can be incorporated. The structure is held together by electronic interactions, there is substantially no covalent bonding between them.

By the term "nanoparticle" it is understood a structure formed by the electrostatic interaction between the chitosan and the hyaluronan and from the ionotropic gelification of said conjugate by means of the addition of an anionic reticulating agent. The electrostatic interaction resulting between the different polymeric components of the nanoparticles and the subsequent reticulating generates characteristic physical entities, which are independent and observable, whose average size is less than 1 µm, i.e. an average size between 1 and 999 nm.

By the term "average size" it is understood the average diameter of the nanoparticle population, which comprises the polymeric reticulated structure, which moves together in an aqueous medium. The average size of these systems can be measured using standard procedures known by a person skilled in the art, and which are described, for example, in the experimental part below.

The nanoparticles of the system of the invention have an average particle size of less than 1 µm, i.e. they have an average size between 1 and 999 nm, preferably between 50 and 500 nm, even more preferably between 100 and 300 nm. The average size of the particles is mainly influenced by the proportion of chitosan with respect to the hyaluronan, by the chitosan deacetylation degree and also by the particle formation conditions (chitosan and hyaluronan concentration, reticulating agent concentration and ratio between them).

The nanoparticles may have a surface charge (measured by zeta potential) which varies depending on the proportion of the chitosan and hyaluronan in the nanoparticles. The contribution to the positive charge is attributed to the amine groups of the chitosan, while the contribution to the negative charge is attributed to the carboxylic groups of the hyaluronan. Depending on the chitosan/hyaluronan proportion, the charge magnitude may vary between - 50 mV and +50 mV.

Frequently, it is of interest that the surface charge is positive in order to improve the interaction between the nanoparticles and biological surfaces, particularly mucous surfaces, which are negatively charged. This way, the biologically active molecule will favourably act on the target tissues. However, in some instances, a neutral charge may be more suitable in order to ensure the stability of the nanoparticles following parenteral administration. The negative charge could also be of interest for the administration to the mucous surface due to the presence of hydrogen bonds, hydrophobic and receptor affinity interactions.

### Chitosan

Chitosan is a polymer of natural origin derived from chitin (poly-N-acetyl-D-glucosamine), where an important part of the acetyl groups of the N have been eliminated by hydrolysis. The degree of deacetylation is preferably greater than 40%, more preferably greater than 60%. In a variant it is between 60-98%. It has an aminopolysaccharide structure and cationic character. It comprises the repetition of n monomeric units of formula (I): 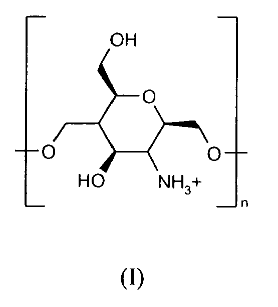 wherein n is an integer, and m units having an acetylated amine group. The sum of n+m represents the degree of polymerization, i.e. the number of monomeric units in the chitosan chain.

The chitosan used to produce the nanoparticles of the present invention has a low molecular weight polymer, understanding as such a chitosan such as described above or a derivative thereof with a molecular weight less than 90 kDa, preferably between 1 and 90 kDa, more preferably between 2 and 50 kDa, even more preferably between 2 and 30 kDa. A range of between about 2 and about 15 kDa is especially preferred. The chitosan with this molecular weight is obtained by methods well known to a skilled person, such as oxidative reduction of the chitosan polymer using different proportions of NaNO₂.

As an alternative to chitosan, a derivative thereof can also be used, understanding as such a chitosan with a molecular weight less 90 kDa wherein one or more hydroxyl groups and/or one or more amine groups have been modified, with the aim of increasing the solubility of the chitosan or increasing the adhesive nature thereof. These derivatives include, among others, acetylated, alkylated or sulfonated chitosans, thiolated derivatives, as is described in Roberts, Chitin Chemistry, Macmillan, 1992, 166. Preferably, when a derivative is used it is selected from O-alkyl ethers, O-acyl esters, trimethyl chitosan, chitosans modified with polyethylene glycol, etc. Other possible derivatives are salts, such as citrate, nitrate, lactate, phosphate, glutamate, etc. In any case, a person skilled in the art knows how to identify the modifications which can be made on the chitosan without affecting the stability and commercial feasibility of the end formulation.

The use of chitosan, or a derivative thereof, with a molecular weight lower than 90 kDa is particularly relevant for the system of the invention because the nanoparticles containing it can be efficiently eliminated or degraded once they are introduced in the biological environment, resulting in a more efficient delivery of the biologically active molecule.

### Hyaluronan

Hyaluronan is a glycosaminoglycan distributed widely throughout connective, epithelial and neural tissues. It is one of the main components of the extracelular matrix and in general contributes significantly to cell proliferation and migration.

Hyaluronan is a linear polymer which comprises the repetition of a disaccharide structure formed by alternate addition of D-glucuronic acid and D-N-acetylglucosamine, linked together via alternating beta-1,4 and beta-1,3 glycosidic bonds as shown in formula (II): 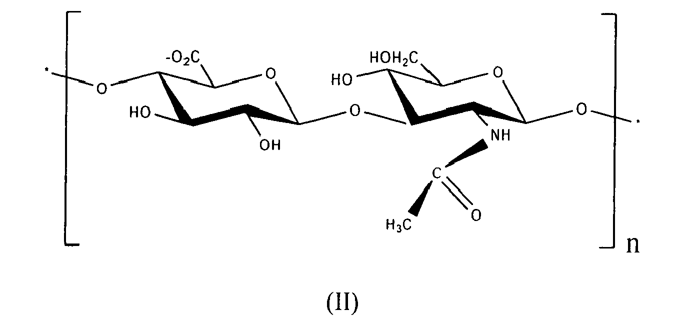 wherein the integer *n* represents the degree of polymerization, i.e. the number of disaccharides units in the hyaluronan chain.

Both sugars are spatially related to glucose which, in the beta configuration, allows all of its bulky groups (the hydroxyls, the carboxylate moiety and the anomeric carbon on the adjacent sugar) to be in sterically favourable equatorial positions while all of the small hydrogen atoms occupy the less sterically favourable axial positions.

The hyaluronan used to produce the nanoparticles of the present invention has a molecular weight comprised between 2 kDa and 160 kDa. In a particular embodiment of the invention the hyaluronan is an oligomer with a molecular weight comprised between 2 and 50 kDa, preferably between 2 and 10 kDa.

The hyaluronan of high molecular weight is commercially available, while that of lower molecular weight can be obtained by fragmentation of hyaluronan of high molecular weight, for example using a hyaluronidase enzyme.

The term "hyaluronan" as used in the present description includes either the hyaluronic acid or a conjugate base thereof (hyaluronate). This conjugate base can be an alkali salt of the hyaluronic acid which include inorganic salts such as, for example, sodium, potassium, calcium, ammonium, magnesium, aluminium and lithium salts, and organic salts such as basic aminoacid salts. In a preferred embodiment of the invention the alkali salt is the sodium salt of the hyaluronic acid.

Hyaluronan is a natural hydrophilic polysaccharide, non-toxic, biodegradable and biocompatible: It has mucoadhesive properties, and it binds specifically to the CD44 receptor present in cell membranes, thus favouring its interaction with cells.

In a variant of the invention, the hyaluronan/chitosan mass ratio in the system is comprised between 2:1 and 1:10, preferably between 2:1 and 1:2 weight:weight. Lower proportions of chitosan would not be recommendable since aggregates or polymers solutions would be obtained.

Irrespective of the nanoparticles composition (chitosan-hyaluronan proportion and molecular weight of hyaluronan, polymer or oligomer), the nanoparticles size is maintained under 1 micrometer, preferably under 500 nm, more preferably under 300 nm. In one embodiment it is below 250 nm, more preferably below 200 nm. This size allows nanoparticles to penetrate epithelial cells, such as corneal epithelial cells, and deliver the biologically active molecule.

The nanoparticles of the system of the invention are formed by ionotropic gelation of the chitosan-hyaluronan system. The reticulating agent is an anionic salt which allows ionic gelation, favouring the spontaneous formation of the nanoparticles. In a preferred embodiment of the invention the reticulating agent is a tripolyphosphate, being more preferred the use of sodium tripolyphosphate (TPP). The reticulating process is very simple and known by the skilled person as described in the background of the invention.

The nanoparticles of chitosan and hyaluronan of the present invention provide systems which have a high capability for associating biologically active molecules, either inside the nanoparticles or adsorbed onto them. Irrespective of the molecular weight of hyaluronan and the chitosan-hyaluronan weight ratio, nanoparticles show efficiencies higher than 90% in associating the active molecules. Therefore, another aspect of the invention relates to a system such as described above which further comprises a biologically active molecule.

The term "biologically active molecule" relates to any substance which is used in the treatment, cure, prevention or diagnosis of a disease or which is used to improve the physical and mental well-being of humans and animals. According to the present invention, the nanoparticles of hyaluronan and chitosan are suitable for incorporating biologically active molecules irrespective of the solubility characteristics thereof. The association capacity will depend on the molecule incorporated, but in general terms it will be high both for hydrophilic molecules and also for those of marked hydrophobic character. These molecules may include polysaccharides, proteins, peptides, lipids, oligonucleotides, polynucleotides, nucleic acids and mixtures thereof. In a preferred embodiment of the invention the biologically active molecule is a polynucleotide, preferably is a DNA-plasmid, such as pEGFP, pBgal and pSEAP.

In another preferred embodiment the biologically active molecule is a polysaccharide such as heparine.

Another object of the present invention is a pharmaceutical composition which comprises the previously defined nanoparticulate system and a biologically active molecule capable of preventing, relieving or curing diseases.

Examples of pharmaceutical compositions include any liquid composition (i.e. suspension or dispersion of the nanoparticles of the invention) for oral, buccal, sublingual, topical, ocular, nasal or vaginal application, or any composition in the form of gel, ointment, cream or balm for its topical, ocular, nasal or vaginal administration.

In a variant of the invention, the composition is for ophthalmic administration. In this case the surface of the nanoparticle is positively charged so that the nanoparticles provide a better absorption of the drugs on the eye surface, via their interaction with the mucous and the surfaces of the corneal epithelial cells which are negatively charged.

The proportion of active ingredient incorporated in the nanoparticles may come to be up to 40% by weight with respect to the total weight of the system. Nevertheless, the suitable proportion will depend in each case on the active ingredient to be incorporated, the indication for which it is used and the efficiency of delivery.

In the specific case of incorporating a polynucleotide such as a DNA plasmid as active ingredient, the proportion thereof in said system would be between 1% and 40% by weight, preferably between 5% and 20%.

When a polysaccharide such as heparin is incorporated, the percentage of this ingredient would be included in the system between 1% and 40%, preferably between 10% and 30%.

An additional object of the present invention consists of a cosmetic composition which comprises the previously defined nanoparticulate system. These cosmetic compositions include any liquid composition (suspension or dispersion of nanoparticles) or any composition which comprises the system of the invention and which is in the form of gel, cream, ointment or balm for its topical administration.

In a variant of the invention, the cosmetic composition may also incorporate active molecules of lipophilic and hydrophilic nature which, although they do not have any therapeutic effect, they have properties as a cosmetic agent. Among the active molecules which may be incorporated in the nanoparticles it can be cited emollient agents, preservatives, fragrance substances, antiacne agents, antifungal agents, antioxidants, deodorants, antiperspirants, antidandruff agents, depigmenters, antiseborrheic agents, dyes, suntan lotions, UV light absorbers, enzymes, fragrance substances, among others.

In another aspect, the present invention relates to a process for the preparation of a system of the invention and which comprises nanoparticles as described above. Said process comprises, on the one hand, the preparation of an aqueous solution of hyaluronan, preferably at a concentration of between 0.1 and 5 mg/mL, and on the other hand, the preparation of an aqueous solution of chitosan, preferably at a concentration of between 0.1 and 5 mg/mL.

The incorporation of the polyanionic salt is performed by dissolution thereof in the aqueous solution of the hyaluronan, preferably at a concentration of between 0.01 and 1.0 mg/mL. Subsequently, both aqueous solutions, one containing the hyaluronan and the anionic salt and the other containing the chitosan, are mixed under stirring, thus obtaining spontaneously the nanoparticles in aqueous suspension.

Optionally, the biologically active molecule is dissolved in the aqueous solution containing the chitosan or in the aqueous solution containing the hyaluronan and the anionic salt, in order to be incorporated inside the nanoparticles.

In another embodiment, the active molecule can be dissolved in the aqueous suspension once the nanoparticles are formed with the aim to be adsorbed on the nanoparticle surface.

In a variant of the process, when the biologically active molecule presents a lipophilic character, it is dissolved, before incorporating it in any of the aqueous solutions previously defined, in a small volume of a mixture of water and a water-miscible organic solvent, such as acetonitrile, preferably in a proportion of about 1:1, which will then be added to one of the aforementioned aqueous solutions, so that the concentration by weight of the organic solvent in the end solution is always less than 10%. In such a case to organic solvent has to be removed from the system, unless it is pharmaceutically acceptable.

The process for preparing the chitosan-hyaluronan nanoparticles of the present invention can further comprise an additional step in which said nanoparticles are lyophilised. From the pharmaceutical point of view it is important to be able to have the nanoparticles available in lyophilised form since this improves their stability during storage and reduces the volumes of product to be manipulated The chitosan-hyaluronan nanoparticles may be lyophilised in the presence of a cryoprotectant, such as glucose, sucrose or trehalose, at a concentration ranging form 1 to 5%. In fact, the nanoparticles of the invention have the additional advantage that the particle size before and after lyophilisation is not significantly modified. That is, the nanoparticles have the advantage that they can be lyophilised and resuspended without any alteration in the characteristics thereof.

The system of the present invention has demonstrated to be a highly efficient carrier, able to interact with epithelial cells and having a great capacity to promote the transfection of a polynucleotide into a cell. The nanoparticles comprised in the system can incorporate into the cell genetic material such as a nucleic acid-based molecule, an oligonucleotide, siRNA or a polynucleotide, preferably a plasmid DNA which encodes a protein of interest, thus making them a potential vehicle in gene therapy. In a particular embodiment, the plasmid DNA is pEGFP or pSEAP.

In vitro studies in three different cell lines, such as HEK293 (Human Embrionary Kidney cell line), HCE (Human Corneal Epithelial cell line) and NHC (Normal Human Conjunctival cell line), as well as *in vivo* studies in the ocular epithelium of some animals, have shown that the nanoparticles comprised in the system of the invention exhibit a very low cell toxicity and that they can be internalized by cells by means of cellular endocytosis processes and also by interaction with specific receptors of the cellular membrane. The subsequent biodegradation of the nanoparticles by biodegradation of hyaluronan and by elimination or biodegradation of chitosan allows the delivery of the DNA-plasmid in a very effective manner, reaching high and long transfection levels, for example with more than 25% of transfected cells for up to 10 days. The best transfection levels were obtained when hyaluronan of low molecular weight (10 kDa) that can be used in the formulation of the nanoparticles. Additionally, this transfection efficiency is also observed when nanoparticles have been subjected to a lyophilization (freeze-drying) process.

Surprisingly, the nanoparticulate system of the invention is also stable at pH between 6.4 and 8.0, notwithstanding that chitosan is not soluble at pHs higher than 6.6-6.8. This makes the nanoparticulate system to be very versatile for different modalities of administration, including nasal and oral administration and topical aplication, and which ensures the stability of the nanoparticles at the plasma pH of 7.4. Additionally, this stability is also of interest for applying the nanoparticles to stable line cell cultures or to stable cultures difficult to transfect "*in vivo*"*.*

Accordingly, an additional object of the invention refers to the use of the system of the present invention in the preparation of a medicament for gene therapy. In a particular embodiment it comprises a polynucleotide comprising a gene capable of functional expression in cells of the patient being treated.

In this sense, some examples of diseases to be treated using the system of the invention are macular degeneration with antisenses against VEGF, epidermolysis bullosa and cystic fibrosis. It is particularly useful for diabetic retinopathy and macular degeneration. It can also be used in the healing of wounds with transient transformation schema.

Finally, due to its high efficiency for transfection, the system and compositions of the invention, containing synthetic or natural polynucleotides, allows their use for transfection of target cells, preferably neoplastic or "normal" mammalian cells, as well as stem cells or cell lines. It is therefore a useful tool for the genetic manipulation of cells. In this sense, the invention is also directed to the use of the system of the invention for the genetic manipulation of cells. Preferably it is for the delivery of nucleic acids, *in vitro* or *ex vivo.*

In one embodiment the nucleic acids are anti-sense oligonucleotides that can specifically base pair to complementary mRNA and prevent mRNA translation and production of the corresponding protein, such as interfering (iRNA) or small interfering RNA (siRNA).

According to a further aspect, the invention relates to the use of the nanoparticles of the invention for incorporation and delivery of nucleic acids. Such delivery is directed to target cells comprising: eukaryotic cells, such as mammalian cells, cell lines, stem cells, primary cell lines, and can lead to transfection or cell transformation in vitro or ex-vivo. Therefore, according to this aspect, the invention relates to a kit for transfection of eukaryotic cells, comprising the nanoparticles of the invention and suitable diluents and/or cell washing buffers.

Below, some illustrative examples are described which reveal the characteristics and advantages of the invention, however, they should not be interpreted as limiting of the object of the invention as is defined in the claims.

### EXAMPLES

As common process to the examples detailed below, the nanoparticles have been characterized from the point of view of size, zeta potential (or surface charge) and encapsulation efficacy.

*Size Distribution* has been performed using photon correlation spectroscopy (PCS; Zeta Sizer, Nano series, Nano-ZS, Malvern Instruments, UK) obtaining average size values of the nanoparticle population.

*The Zeta potential* has been measured using Laser Doppler Anemometry (LDA; Zeta Sizer, Nano series, Nano-ZS, Malvern Instruments, UK). To determine the electrospheric mobility, the samples were diluted in Milli-Q water.

The *association efficiency* was evaluated by electrophoresis gel and by PicoGreen®, which allows the exact quantification of free p-DNA.

The chitosan (Protasan UP Cl 113) used in the examples is from NovaMatrix-FMC Biopolymer. This chitosan is subjected to an oxidative reduction using NaNO₂ at different weight ratios (CS/NaNO₂ 0.01; 0.02; 0.05; 0.1) in order to get chitosan with low molecular weights of 11, 14, 31 and 45 kDa. These values were determined by SEC "Size Exclusion Chromatography" with a Light Scattering detector.

The hyaluronan used in the examples is the sodium salt of hyaluronic acid. The hyaluronan with molecular weight of 160 kDa is from Bioibérica S.A., and that with molecular weight of less than 10 kDa is obtained by fragmenting the hyaluronan of 160 kDa with hyaluronidase and passing the solution through a 10 kDa filter.

Sodium tripolyphosphate is from Sigma Aldrich, Co., DNA-plasmids pEGFP, pBgal and pSEAP from Elim. Biopharmaceutical Corp. and the remaining products used come from Sigma Aldrich.

The following abbreviations have been used in the examples:
CS: chitosan with molecular weight of 125 kDa
CSO: chitosan with low molecular weight of 10-12, 14, 31 and 45 kDa
HA: sodium hyaluronate with molecular weight of 160 kDa
HAO: sodium hyaluronate with low molecular weight of less than 10 kDa
TPP: sodium tripolyphosphate
PBS: phosphate buffer saline
HBSS: hans balance salt solution

### Example 1.

### Nanoparticles' preparation.

Nanoparticles made of chitosan (CSO, with different molecular weights 11, 14, 31 and 45 kDa) and sodium hyaluronate (HA or HAO) in different proportions, with DNA-plasmid (pEGFP, pBgal or pSEAP) incorporated therein, were obtained by the ionotropic gelification process.
Two aqueous solutions were prepared, one containing 0.625 mg/mL of CSO in milli-Q water and the other containing 0.625 mg/mL of HA or HAO, 0.025 mg/mL of TPP and the corresponding plasmid DNA in a proportion varying from 5 to 20% by weight.
For the nanoparticles containing a proportion 1:2 of hyaluronate:chitosan, 0.75 ml of the first aqueous solution were mixed with 0.375 ml of the second solution under magnetic stirring, thus obtaining spontaneously the nanoparticles suspended in water.
For the nanoparticles containing a proportion 1:1 of hyaluronate:chitosan, 0.75 ml of the first aqueous solution were mixed with 0.75 ml of the second solution under magnetic stirring, thus obtaining spontaneously the nanoparticles suspended in water.
For the nanoparticles containing a proportion 2:1 of hyaluronate:chitosan, 0.75 ml of the first aqueous solution were mixed with 1.5 ml of the second solution under magnetic stirring, thus obtaining spontaneously the nanoparticles suspended in water.
For comparative studies, nanoparticles containing CS (with molecular weight of 125 kDa) instead of CSO were also prepared following the same procedures as described above using the same proportions of chitosan and hyaluronate.
As can be noted in table I, the nanoparticles size is maintained under 250 nm. Nevertheless, the zeta potential values are dependent on the chitosan and hyaluronate proportion, being less positive while increasing the hyaluronate content in the nanoparticles (table I).

**Table I: Size and Z potential values of nanoparticles as a function of chitosan molecular weight**

| | CS/CSO:HA weight ratio | | | | | |
|---|---|---|---|---|---|---|
| | 2:1 | | 1:1 | | 1:2 | |
| | size (nm) | Z (mV) | size (nm) | Z (mV) | size (nm) | Z (mV) |
| CS 125 kDa | 163 | +25,3 | 160 | +17,7 | 191 | -29,9 |
| CSO 45 kDa | 203 | +25.5 | 256 | +25.8 | 156 | -13.3 |
| CSO 31 kDa | 158 | +24.2 | 147 | +21.8 | 156 | -11.3 |
| CSO 14 kDa | 122 | +20.4 | 176 | +8.7 | 146 | -3.3 |
| CSO 11 kDa | 173 | +23.4 | 103 | +9.9 | 123 | -2.8 |

### Example 2

### Encapsulation efficiency assays and sustained in vitro release

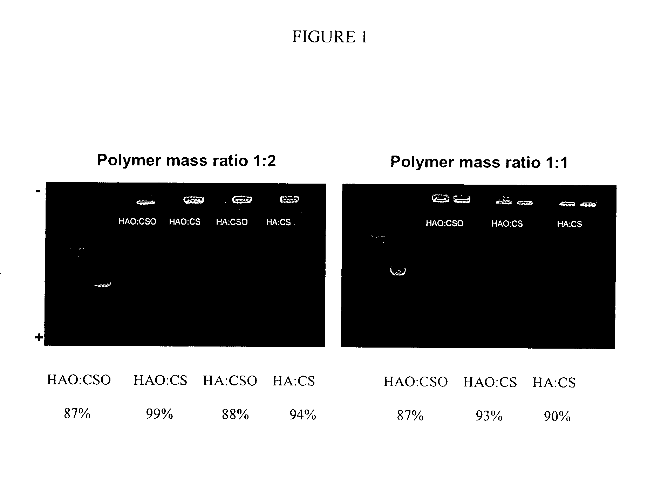

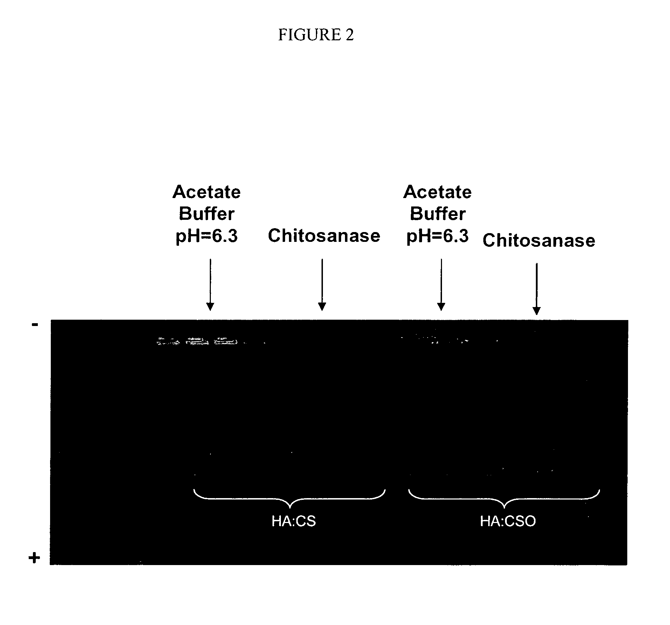

The nanoparticles obtained according to the procedure described in example 1 (either those containing CSO with 10-12 kDa or CS) were incubated at 37°C in a buffered medium under stirring for a period enough to permit the release of the plasmid. The released quantity is assessed at different times by electrophoresis gel.
It was observed (figure 1), that no release of plasmid is produced when the nanoparticles are incubated in acetate buffer. Additionally, irrespective of the molecular weight of hyaluronate and the chitosan-hyaluronate weight ratio, nanoparticles show association efficiencies higher than 85%.
It is also possible to add specific enzymes for the degradation of the polymers constituting the nanoparticles matrix (e.g. chitosanase, 0.105 U enz/170 µL formulation) in order to degrade the nanoparticulate system and thus facilitating the release of encapsulated molecule. It can be observed (figure 2) that after degradation of the nanoparticles, the plasmid is totally released in less than 1 hour.

### Example 3

### In vitro citotoxicity analysis

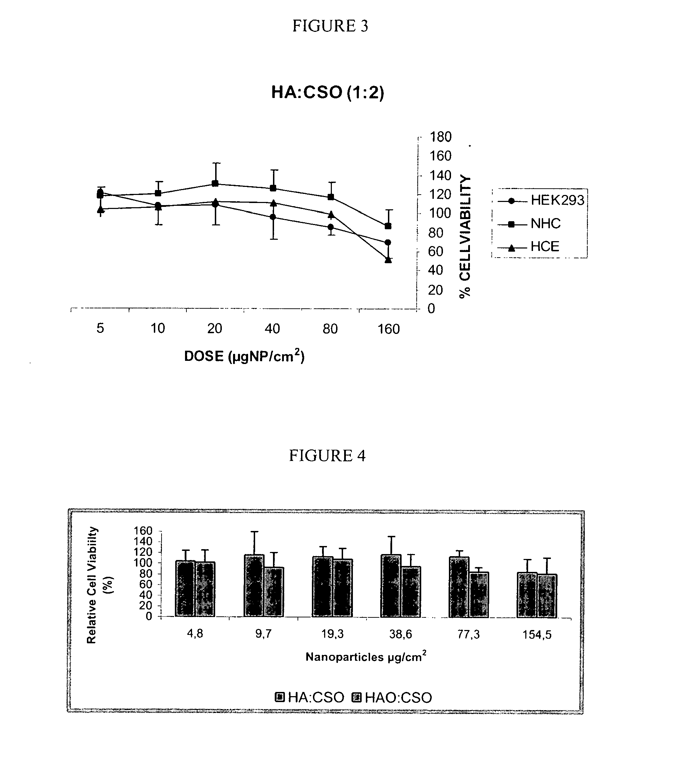

From the nanoparticles suspensions obtained in example 1, specifically those containing chitosan of low molecular weight of 10-12 kDa, different serially solutions were prepared with the aim of having different nanoparticles concentrations in order to evaluate the cellular viability as a function of the doses.
This study was performed in three different cell lines:
- HEK293 (Human Embrionary Kidney cell line)
- HCE (Human Corneal Epithelial cell line)
- NHC (Normal Human Conjunctival cell line).
A MTS assay is performed, wherein the cellular viability is evaluated respect to the 100% (which is considered the culture where only culture medium has been added). The cells must be plated the previous day of the experiments in a quantity of 300.000 cells per well. The culture medium is then taken in and the cell culture washed twice with PBS. After that, the nanoparticles suspension was added to the cell culture and HBSS is added until completing a volume of 1000 µL. The cells are incubated for 1 hour at 37°C and then washed with HBSS or PBS. Subsequently, the MTS reactive (reactive composed of solutions of a novel tetrazolium compound; 3-(4,5-dimethylthiazol-2-yl)-5-(3-carboxymethoxyphenyl)-2-(4-sulfophenyl)-2H-tetrazolium, inner salt) is added (120 µL/well) and the plate is read at 490 nm after 3 h. The reactive is prepared at the moment to be used.
The doses of nanoparticles assayed were 160, 80, 40, 20, 10 and 5 µg/cm².
It was observed [figure 3] that citotoxicity levels are dependant on the cell line. In general terms, the nanoparticles of the invention exhibit a very low toxicity [figure 4].

### Example 4

### In vitro studies of transfection efficiency of nanoparticles.

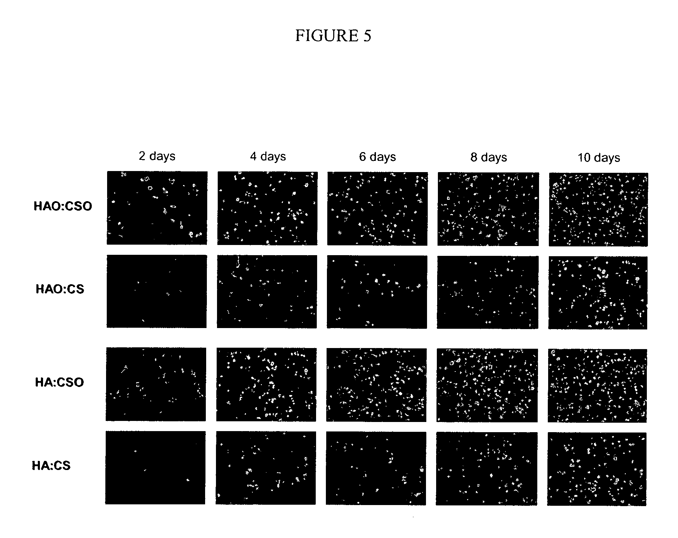

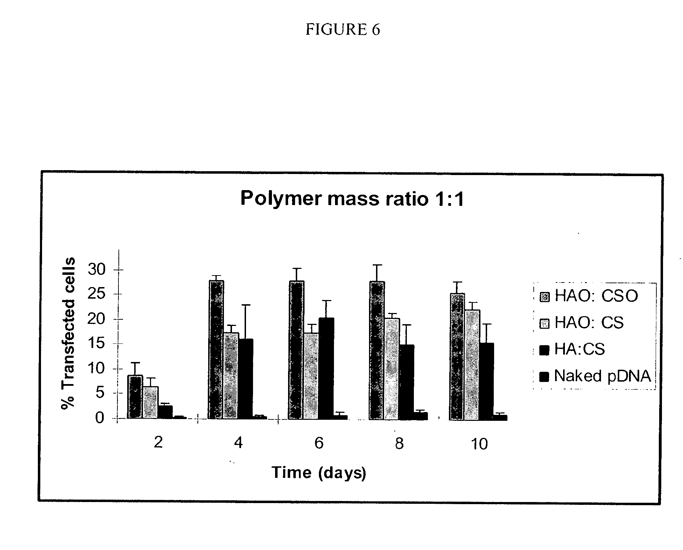

In order to prove the transfection efficiency of the nanoparticulate system, in vitro studies were performed in the same cell lines used in example 3.
Nanoparticles containing CSO of 10-12 kDa and HA or HAO were prepared according to the process previously described in example 1. For comparative studies, nanoparticles containing CS (M_{w}=125 kDa) were also used.
The cells must be plated 24 h before starting the experiments in a quantity of 300.000 cells per well. The culture medium is then taken in and the cell culture washed with PBS for twice. After that, 300 µL of HBSS were added per well. Subsequently, the nanoparticles suspension was added in such a quantity that 1 µg pDNA/well was added. The cells are incubated for 5 hour at 37°C, then washed with HBSS or PBS and more culture medium was added. This culture medium is changed the following day and every time before the measure of expression levels.
The protein expression levels were quantified from the second day post-transfection, and every 2 days during a period of 10 days. The quantification is performed with fluorescence microscopy and subsequent treatment of the images by means of Photoshop Elements.
It can be observed from figures 5 and 6 that the nanoparticles containing chitosan of low molecular weight (CSO: 10-12 kDa) exhibit a higher and longer lasting transfection levels with more than 25% of transfected cells for up to 10 days, when comparing to nanoparticles containing chitosan with a molecular weight of 125 kDa.

### Example 5

### In vitro cell uptake analysis

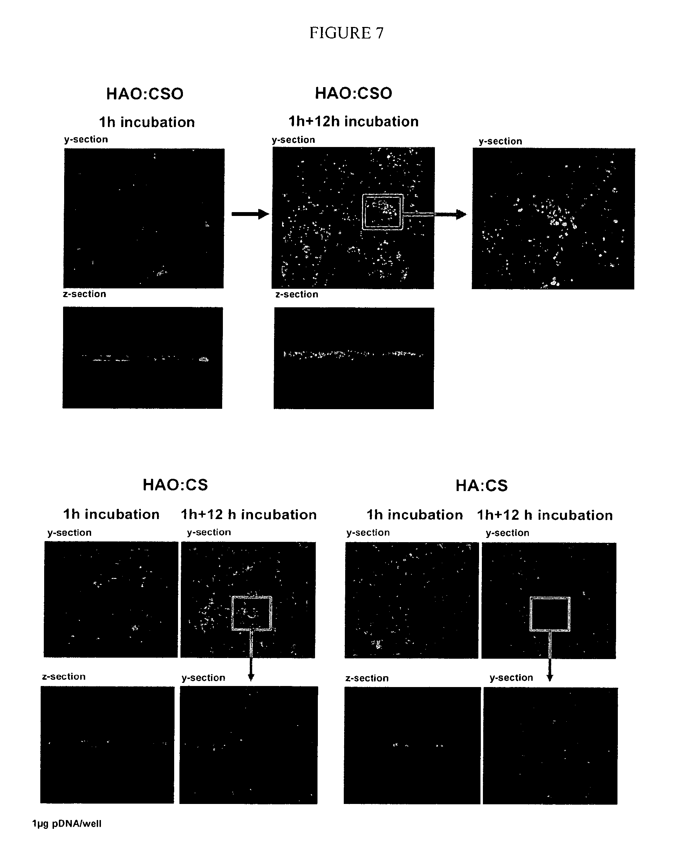

In order to prove the internalization of the nanoparticulate into a cell system, in vitro studies were performed using the cell line HEK293.
The nanoparticles were prepared in such a way that they can be visualized in confocal microscope. Thus, the sodium hyaluronate was previously labelled with fluoresceine. The following nanoparticles formulations were analyzed: HAO:CSO [10-12 kDa]; HAO:CS and HA:CS in a proportion 1:2, loaded with 1% of plasmid.
300.000 cells/well were plated 24 hours before the experiment. Subsequently, the culture medium is aspirated, then washed with PBS and finally the nanoparticles are added to the culture, completing with HBSS until a volume of 200 µL is obtained.
The cell culture is incubated for 1 or 2 hours. After cell incubation, the cell nuclei are stained with propidium iodine and the samples are prepared in order to be observed at the confocal microscope.
As can be seen in figure 7, after 1 hour post-incubation, the fluorescence is observed in the cellular cytoplasm irrespective of nanoparticle formulation, so that the nanoparticles were effectively internalized by cells. The labelled nanoparticles are localized in vacuoles at the intra-cellular and peri-nuclear levels, when the cell culture is treated with low molecular weight (10-12 kDa) chitosan nanoparticles [CSO:HAO]. This intracellular localization points-out the potential of these nanoparticles as intracellular delivery carriers for nucleic acid-based biomolecules.

### Example 6

### Nanoparticles interaction with CD44 receptor

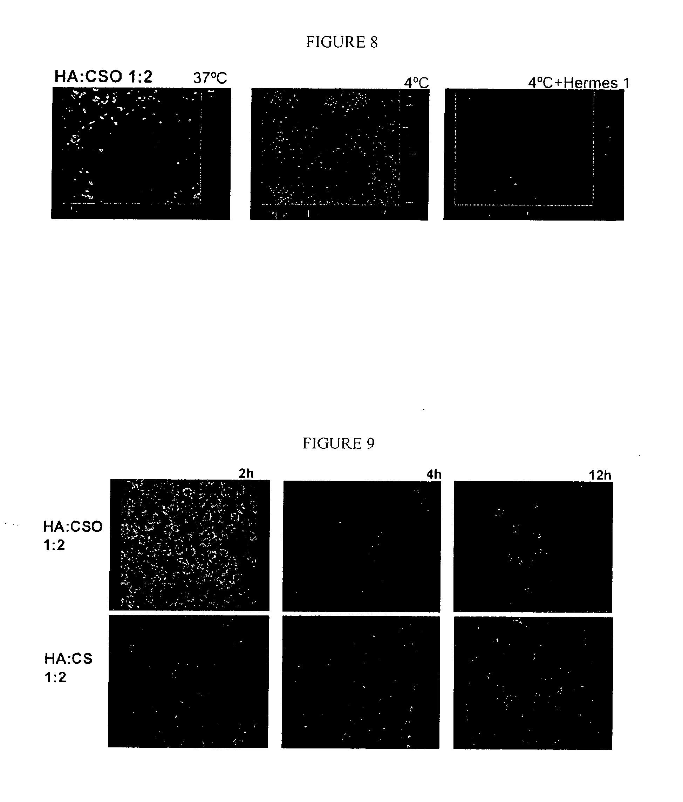

In this example it is evaluated if the nanoparticles are able to be internalized after interaction with the receptor CD44, a specific receptor for hyaluronan. Nanoparticles are prepared according to example 1, except that hyaluronate is previously labelled with fluoresceinamine. The formulation was HA:CSO [10-12 kDa] in a proportion 1:2.
In this study the cell line HCE was used, the cells being incubated at different temperatures (4 and 37°C). As can be seen in figure 8 a) and b), the nanoparticles are internalized by the cells since the plasmid and the hyaluronate can be observed at intracellular level. At 37°C the nanoparticles are internalized by endocytosis and/or by interaction with CD44, while at 4°C, these nanoparticles are solely internalized if they interact with receptor CD44.
Additionally, in one of the experiments the receptor CD44 is blocked with Ab Hermes 1. As shown in figure 8 c), at 4°C it can be observed that nanoparticles are not internalized, since as we have mentioned before, at this temperature the internalization is only due to the interaction with the receptor.

### Example 7

### In vivo studies of the efficiency of nanoparticles to interact with ocular epithelial cells and to be degraded inside the cells

In order to evaluate the interaction of nanoparticles with ocular mucous, nanoparticles comprising a formulation of HA:CSO [10-12 kDa] and HA:CS in a proportion 1:2 were prepared according to example 1, except that the hyaluronate was previously labelled with fluoresceine (HA-fl). A solution of HA-fl was used as a control. The nanoparticles were concentrated by centrifugation and subsequently resuspended in milliQ water, being the nanoparticle concentration 3 mg/mL. 0.3 mg of nanoparticles was instilled into the eye of rabbits of 2 kg. Four instillations of 25 µL were performed every 10 minutes.
Animals were sacrificed after 2, 4 or 12 hours post-instillation and then the cornea and conjunctiva dissected. The fresh tissues were observed at the confocal microscope.
As can be seen in figure 9, intense fluorescence signals corresponding to the nanoparticles were detected inside the cells of the cornea and the conjunctiva. In addition, it was observed that fluorescence intensity decreases over the time, being more evident for the nanoparticles comprising chitosan of molecular weight 10-12 kDa. This suggests that somehow these nanoparticles are assimilated or degraded inside the cells. It is known that hyaluronidase is responsible for the degradation of hyaluronan in the ocular tissues. However, these results also state that the chitosan of low molecular weight is also degraded at the intracellular level with high efficiency which demonstrates the advantage of using the chitosan with molecular weight of less than 100 kDa, in order to achieve an efficient delivery of biologically active molecules inside the epithelial cells.

### Example 8

### In vivo studies of the efficiency of nanoparticles to transfect ocular tissues

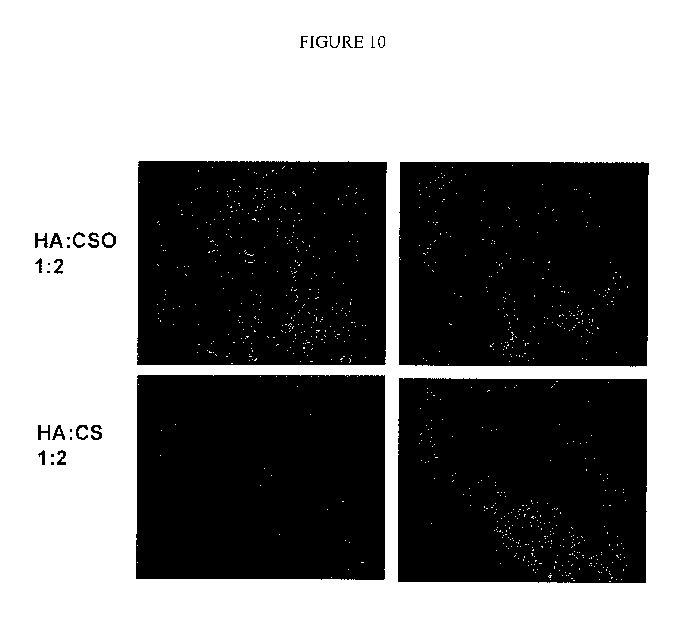

With the aim to determine the ability of the nanoparticles of the invention to transfect ocular tissues in vivo and to determine the efficacy of these carriers for ophthalmic gene delivery, nanoparticles prepared according to the procedures described in example 7 loaded with 10% pGFP were topically administered to normal conscious rabbits of 2 kg in the following doses: 25, 50 and 100 µg pGFP/eye. 15 µL of the formulation were administered every 10 minutes (for the higher dose 50+50 µL were administered leaving an intermediate period of 30 minutes in order to avoid the formulation drain). Animals were sacrificed and then the cornea and conjunctiva dissected. The expression of the encoded green protein was observed in the excised corneal and conjunctival epithelia after 2, 4 and 7 days post-transfection at the confocal microscope. As can be observed in Figure 10, the nanoparticles containing chitosan of low molecular weight (CSO) yield higher transfection levels at lower doses, and hence were used to evaluate the duration of the gene expression. At 4 and 7 days post-transfection green corneal cells were still photographed.
These results were further corroborated by evaluation of the gene expression in enucleated eyes of Sprague-Dawley rats of 250 g. In this case the same nanoparticles formulations were used except that pβgal was loaded in the nanoparticulate system in a proportion of 10%.
2.5 µg of nanoparticles in a volume of 5µL were administered to the rats and after 48 hours the animals were sacrified and the eyes enucleated, fixed in paraformaldehide and finally subjected to staining with X-gal in order to visualize the expression of the protein.

These results provide evidence of the capacity of nanoparticles made of chitosan of low molecular weight and hyaluronan to enter the corneal epithelial cells and to be degraded inside these cells, thus delivering DNA-plasmid in a very effective manner and reaching important transfection levels. Therefore, these nanoparticles may represent a new strategy for gene therapy, in this particular case for the treatment of several ophthalmic diseases.

### Example 9

### Lyophilization of nanoparicles of chitosan and hyaluronic acid

Nanoparticles obtained according to example 1, specifically those containing CSO:HA and CSO:HAO, in a weight proportion 2:1 and loaded with 7% of plasmid respect to the total polymer weight, were subjected to a lyophilization (freezed-drying) process, by adding different crioprotectors (sacarose, trehalose and glucose at 1 and 5% by weight) and cooling to -80°C, (first and second desecation at -50°C). Subsequently, the formulations were resuspended in water and the particle size was measured. As shown in table II, all the formulations were resuspended suitably leading to the initial nanoparticulate system.

**Table II: Nanoparticles size after lyophilization in the presence of cryoprotectors (sacarose, trehalose and glucose) and resuspended in water (n=2). Molecular weight chitosan: 11 kDa; weight ratio CSO:HA 2:1**

| nanoparticulate system | cryoprotector | amount cryoprotector (%; p/v) | size after freeze-drying process (nm) |
|---|---|---|---|
| | sucrose | 5% | 156 |
| | | 1% | 174 |
| CSO 11 kDa-HA | trehalose | 5% | 129 |
| | | 1% | 135 |
| | glucose | 5% | 152 |
| | | 1% | 160 |
| | sucrose | 5% | 131 |
| | | 1% | 170 |
| CSO 11 kDa-HAO | trehalose | 5% | 148 |
| | | 1% | 121 |
| | glucose | 5% | 131 |
| | | 1% | 189 |

Later, another lyophilization study was performed but using chitosan with different molecular weights in order to know how affect the molecular weight in the lyophilization and resuspension of nanoparticulate systems. In this case the hyaluronate used has a molecular weight of 160 kDa and the proportion CSO:HA was 1:2 and 2:1.

**Table III: Nanoparticles size after lyophilization in the presence of glucose and resuspended in water (n=2). Molecular weight chitosan: 11, 14, 31 and 45 kDa; weight ratio CSO:HA 2:1 and 1:2**

| nanoparticulate system | weigth ratio CSO:HA | size after freeze-drying process (nm) |
|---|---|---|
| CSO 45 kDa-HA | 2:1 | 162 |
| | 1:2 | 312 |
| CSO 31 kDa-HA | 2:1 | 210 |
| | 1:2 | 203 |
| CSO 14 kDa-HA | 2:1 | 249 |
| | 1:2 | 233 |
| CSO 11 kDa-HA | 2:1 | 233 |
| | 1:2 | 234 |

As shown in table III, irrespective of the chitosan molecular weight (11, 14, 31 or 45 kDa) and CSO:HA weight proportion (2:1 or 1:2), all systems are suitably resuspended maintaining particle size in a nanometric range.

### Example 10

### Nanoparticles stabilization at different pH

After the lyophilization and resuspension of nanoparticulate systems containing CSO:HA and CSO:HAO (Mw CSO: 11 kDa), these were diluted in buffers at different pHs.

As can be seen in table IV, the stability of the nanoparticulate systems varies depending on the composition of each system. For example, nanoparticles containing CSO:HA are stable at pH 7.4 and 8.0, whilst nanoparticles containing CSO:HAO are stable at pH 6.4 and 8.0. However, it is surprising that both systems are stable at pH 8.0, since it is known that chitosan is not soluble at pH higher than 6.6-6.8. Consequently, the nanoparticulate systems are versatile for the administration of DNA due to their stability at different pHs.

**Table IV: Nanoparticles size after lyophilization in the presence of cryoprotectors (sacarose, trehalose and glucose), resuspended in water and stored at 37°C in buffers at different pH 6.4; 7.4 and 8.0.**

| nanoparticulated system | cryoprotector | pH 7.4 | | pH 6.4 | | pH8.0 | |
|---|---|---|---|---|---|---|---|
| | | initial size (nm) | 30 min (nm) | initial size (nm) | 30 min (nm) | initial size (nm) | 30 min (nm) |
| CSO 11 kDa-HA | sucrose 5% | 311 | 385 | 1012 | 3885 | 246 | 267 |
| | trehalose 5% | 347 | 744 | 975 | 4760 | 217 | 252 |
| | glucose 5% | 267 | 314 | 1288 | 4319 | 221 | 308 |
| CSO 11 kDa- HAO | sucrose 5% | 798 | 1482 | 134 | 191 | 361 | 501 |
| | trehalose 5% | 943 | 1242 | 168 | 154 | 378 | 473 |
| | glucose 5% | 500 | 1148 | 131 | 129 | 285 | 302 |

Additionally, the stability of the nanoparticles was analyzed using different CSO:HA proportions (2:1, 1:1, 1:2) selecting chitosan with a molecular weight of 45 kDa. As shown in table V, irrespective of CSO:HA weight proportion, the nanoparticles are stable at pH 7.4 and 8.0.

**Table V: Nanoparticles stability in buffers at pH 7.4 and 8.0 after lyophilization and incubation at 37°C (n=2)**

| nanoparticulate system | pH buffer | time (min) | CSO:HA weight ratio | | |
|---|---|---|---|---|---|
| | | | 2:1 | 1:1 | 1:2 |
| | | | size (nm) | size (nm) | size (nm) |
| CSO 45 kDa- HA | 7.4 | 0 | 471 | 388 | 276 |
| | | 30 | 492 | 472 | 481 |
| | 8.0 | 0 | 477 | 386 | 367 |
| | | 30 | 541 | 512 | 322 |

Also, as can be seen in table VI, nanoparticles size is maintained in the nanometric range during at least 1 week at pH 8.

**Table VI: Nanoparticles stability in phosphate buffer at pH=8.0 (n=2)**

| nanoparticulate system | CSO:HA weight ratio | pH 8.0 | |
|---|---|---|---|
| | | initial size (nm) | size after I week (nm) |
| CSO 45 kDA-HA | 2:1 | 203 | 477 |
| | 1:1 | 256 | 386 |
| | 1:2 | 156 | 322 |

### Example 11

### In vitro transfection studies of lyophilized nanoparticles in cell line HEK293

In order to know if our nanoparticles systems, after their lyophilization, have capacity to transfect cell cultures, different formulations of CSO and HA loaded with 7% of plasmid pEGFP, were analyzed in the cell line HEK293 (dose: 1 µg pEGFP). The molecular weights of the chitosan used in this example were 14, 31 and 45 kDa and weight proportion of CSO:HA was 2:1 and 1:2. These formulations were previously lyophilized in the presence of glucose at 1% (w/V) and resuspended.

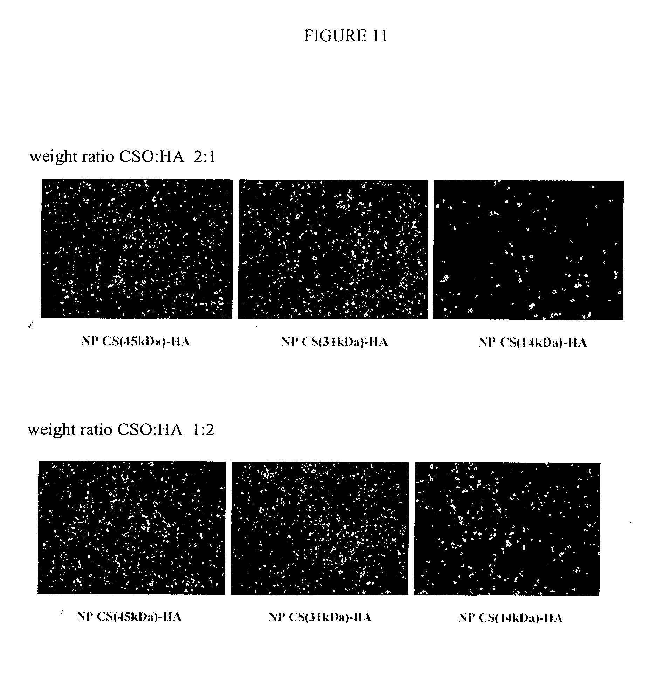

The images taken from fluorescence microscope (figure 11) showed intracellular presence of fluorescence protein, and consequently they pointed out that lyophilized nanoparticulate systems containing CSO and HA present capacity to transfect. The photos were taken 4 days after putting in contact the formulations with the cells.

## Claims

1. A system for the release of biologically active molecules which comprises nanoparticles with an average size less than 1 micrometer, wherein the nanoparticles comprise:
a) hyaluronan or a salt thereof; and
b) chitosan or a derivative thereof,
**characterized in that** the molecular weight of the chitosan or the derivative thereof is less than 90 kDa.

2. System according to claim 1 wherein the molecular weight of the chitosan or a derivative thereof is comprised between 1 and 90 kDa, more preferably between 2 and 50 kDa, even more preferably between 2 and 15 kDa.

3. System according to claim 1 wherein the molecular weight of the hyaluronan or its salt is comprised between 2 and 160 kDa.

4. System according to any of claims 1 to 3 wherein the hyaluronan/chitosan mass ratio is comprised between 2:1 and 1:10, preferably between 2:1 and 1:2 w:w.

5. System according to any of claims 1 to 4 wherein the hyaluronan is the sodium salt of hyaluronic acid.

6. System according to any of claims 1 to 5 wherein the nanoparticles are reticulated by means of a reticulating agent.

7. System according to claim 6 wherein the reticulating agent is a tripolyphosphate, preferably sodium tripolyphosphate.

8. System according to any of claims 1 to 7 wherein the nanoparticles have an average size between 1 and 999 nm, preferably between 50 and 500 nm, more preferably between about 100 and about 300 nm.

9. System according to any of claims 1 to 8 which further comprises a biologically active molecule selected from the group consisting of polysaccharides, proteins, peptides, lipids, oligonucleotides, polynucleotides, nucleic acids and mixtures thereof.

10. System according to claim 9 wherein the biologically active molecule is a nucleic acid-based molecule, an oligonucleotide, siRNA or a polynucleotide, preferably it is a DNA-plasmid.

11. Pharmaceutical composition which comprises a system such as defined in any of claims 1 to 10 and a biologically active molecule capable of preventing, relieving or curing diseases.

12. Composition according to claim 11 in a form suitable for ophthalmic administration.

13. A process for the preparation of a system according to any of claims 1 to 10, which comprises:
a) preparing an aqueous solution of hyaluronan or a salt thereof;
b) preparing an aqueous solution of chitosan or a derivative thereof;
c) adding a reticulating agent to the solution of the hyaluronan or its salt, and
d) mixing under stirring solutions obtained in b) and c) with spontaneous formation of the nanoparticles;

14. A process as defined in claim 13 wherein a biologically active molecule is dissolved in any of the previous aqueous solutions b) or c) before the formation of the nanoparticles,

15. A process as defined in claim 13 wherein a biologically active molecule is dissolved in the nanoparticles suspension obtained in step d).

16. Use of a system as defined in any of claims 1 to 10 in the preparation of a medicament for gene therapy.
